# EUROPEAN PATENT SPECIFICATION

(11) **EP 4 424 346 B1**
(45) Date of publication and mention of the grant of the patent: **14.01.2026**
(21) Application number: 23207251.2
(22) Date of filing: 01.11.2023
(51) Int. Cl.: A61M 11/02, A61M 13/00

(54) **DISPENSER DEVICE FOR SPRAYING POWDER**
SPENDERVORRICHTUNG ZUM ZERSTÄUBEN VON PULVER
DISPOSITIF DISTRIBUTEUR POUR PULVÉRISER DE LA POUDRE

(30) Priority: 03.03.2023 KR 20230028780
(43) Date of publication of application: 04.09.2024
(60) Divisional application: 25216196.3
(73) Proprietor: Nextbiomedical Co., Ltd., Incheon 22013 (KR)
(72) Inventor: LEE, Donhaeng, 04425 Seoul (KR); LEE, Eunhye, 21982 Incheon (KR); KIM, Seyun, 22764 Incheon (KR)
(74) Representative: J A Kemp LLP

(56) References cited:
- GB-A- 2 520 324
- US-A1- 2003 181 917
- US-A1- 2013 218 072
- US-A1- 2014 207 097
- US-A1- 2019 232 030
- US-A1- 2020 100 986
- US-A1- 2020 101 476
- US-A1- 2021 205 543

## Description

### BACKGROUND OF THE INVENTION

### 1. Field of the invention

The present disclosure relates to relates to a dispenser device for spraying powder and, more specifically, to a dispenser device for spraying powder, improved to prevent a catheter from bending, to prevent fine spray phenomenon of powder, and to eliminate the flow of powder during spraying.

### 2. Description of the Prior Art

Gastrointestinal bleeding is a clinical disorder commonly encountered in clinical practice, and more than 80% of gastrointestinal bleeding occurs in the upper gastrointestinal tract. Upper gastrointestinal bleeding is a disorder in which blood is vomited or a bloody stool is caused by bleeding from lesions in the esophagus, stomach, and duodenum. In upper gastrointestinal bleeding, more than 90% of bleeding sites can be identified through endoscopy, and 40-50% are known to be caused by gastric or duodenal ulcer bleeding. Recently, polypectomy of the stomach or large intestine, mucosal resection for the treatment of early gastric cancer and colorectal cancer, and therapeutic endoscopic procedures have been widely performed. However, bleeding during or after these procedures requires emergency surgery or even leads to death. Hemostasis using an endoscope is used to treat bleeding during surgery, gastrointestinal bleeding, and bleeding occurring during tissue collection for biopsy. The hemostasis using an endoscope is a method of hemostasis by bringing an endoscopic conduit (catheter) inserted into the body close to a damaged mucous membrane requiring hemostasis, and then administering and spraying a hemostatic agent in powder form through the conduit. To this end, various types of pharmaceutical powder sprayer have been developed.

US 2019/232030A discloses an apparatus for delivering a powdered agent into a subject's body which includes a first passage for receiving a pressurized gas. The apparatus also may include a container housing a powdered agent. The container may be in fluid connection with the first passage. At least a portion of the pressurized gas is introduced into the powdered agent in the container to fluidize the powdered agent. The apparatus also may include a second passage for receiving the powdered agent from the container. In a first configuration of the apparatus, the second passage may not be in fluid connection with the container. In a second configuration of the apparatus, the second passage may be in fluid connection with the container. The apparatus may be configured to transition between the first configuration and the second configuration.

US 2003/181917A discloses surgical devices and methods to apply bioactive agents to surgical sites. The devices include, e.g., a body for attachment of a pressurized fluid vessel and a reservoir of bioactive agent for controlled spraying of an aerosol of bioactive agent.

However, the powder spraying device developed to date has the following problems: even in a situation where powder does not need to be sprayed, a fine spray phenomenon in which powder is sprayed finely is observed, resulting in clogging of the end of a catheter conduit; and in the process of operation after attaching a thin catheter to the powder spraying device, the catheter is bent, causing powder to be improperly sprayed or a conduit of the catheter to be damaged. In addition, there is a user requirement that a function enabling a user to stop and respray powder according to his/her will during a procedure is required. Accordingly, there is a need to develop an improved form of a dispenser device for spraying pharmaceutical powder.

### SUMMARY OF THE INVENTION

As described above, the present inventors have made intensive research efforts to develop an improved form of dispenser device for spraying pharmaceutical powder to solve the above problems. As a result, the present disclosure was completed by introducing a tube cap in the shape of a fallopian tube and introducing a rotary valve for opening and closing to completely control the flow of pharmaceutical powder.

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

Accordingly, an aspect of the present disclosure is to provide an improved dispenser device for spraying pharmaceutical powder, including the above-described configuration.

Another aspect of the present disclosure is to provide a method of using the dispenser device for spraying pharmaceutical powder, described above.

The present disclosure relates to a dispenser device for spraying pharmaceutical powder, including the above-described configuration, and the dispenser device can prevent a catheter from bending and spray pharmaceutical powder without fine spray of the powder.

### BRIEF DESCRIPTION OF THE DRAWINGS

The above and other aspects, features, and advantages of the present disclosure will be more apparent from the following detailed description taken in conjunction with the accompanying drawings, in which:
FIG. 1A is a perspective view of a dispenser device for spraying pharmaceutical powder according to an embodiment of the present disclosure.
FIG. 1B is an exploded view of the dispenser device for spraying pharmaceutical powder, illustrating each component thereof according to an embodiment of the present disclosure.
FIG. 2 is a cross-sectional view of a housing according to an embodiment of the present disclosure.
FIG. 3 illustrates a structure in which the diameters of both ends of a flow path in a valve are wider than the diameter of the middle of the flow path to prevent the valve of the present disclosure from becoming inoperable.
FIG. 4 illustrates a fine protrusion formed to block fine gaps in a portion (a valve rotation part) where a control valve is combined with the housing in the device for spraying pharmaceutical powder according to the present disclosure.
FIG. 5 is an enlarged view of a portion where the valve and the fine protrusion of a valve accommodating part are in contact with each other.
FIG. 6 is a perspective view and a cross-sectional view illustrating a combination of a drug container adapter, a drug container, and the housing according to the present disclosure.
FIG. 7 illustrates the shape of a tube cap configured to guide an existing conduit 20 in general use to a conduit mounting part.
FIG. 8 illustrates a tube cap configured to guide the conduit 20 to the conduit mounting part in order to prevent the conduit 20 from bending, according to the present disclosure.
FIG. 9 illustrates the shape of a bottom cap 500 having a groove formed on the bottom surface thereof so that pharmaceutical powder may be collected at a portion where first and second transfer tubes of the present disclosure meet and communicate with each other.
FIG. 10 illustrates an air supply device additionally coupled to the dispenser device for spraying pharmaceutical powder according to the present disclosure.
FIG. 11, which illustrates a dispenser device for spraying pharmaceutical powder in which a first transfer tube and a control valve of the present disclosure have two separate channels, respectively, is a cross-section view of the control valve in an open state.
FIG. 12, which illustrates a dispenser device for spraying pharmaceutical powder in which the first transfer tube and the control valve of the present disclosure have two separate channels, respectively, is a cross-sectional view of the control valve in a closed state.
FIG. 13 illustrates a three-dimensional structure and a cross section of the control valve of FIG. 12, which has two separate channels and is in a closed state.

### DETAILED DESCRIPTION OF THE EXEMPLARY EMBODIMENTS

Hereinafter, the present disclosure will be described in more detail through embodiments of the present disclosure with reference to the accompanying drawings. However, these embodiments are only for explaining the present disclosure in more detail, and the present disclosure may be implemented in many different forms. It will be apparent to those skilled in the art that the scope of the present disclosure is not limited by these embodiments according to the gist of the present disclosure, and the present disclosure is only defined by the claims to be described later.

In addition, terms used in the present disclosure are only used to describe specific embodiments, and are not intended to limit the present disclosure. Singular expressions include plural expressions unless the context clearly dictates otherwise. In the entire specification, "including" a certain element means that other elements may be further included without excluding other elements unless otherwise stated.

In the present specification, the term "dispenser device for spraying pharmaceutical powder" will be described as an example of a type of sprayer that separately includes an air supply device and combines the same with the dispenser device for spraying pharmaceutical powder. However, the "dispenser device for spraying powder" intended to be encompassed in the present specification means a part or all of a device having a purpose for spraying powder. Therefore, the dispenser device for spraying powder of the present disclosure may mean some components of a powder sprayer, or an entire finished product including the same, and if the dispenser device for spraying powder is a product having elements described with reference to the claims and drawings of the present specification, it may mean not only the form of a dispenser device for spraying powder presented as some components in an embodiment of the present specification, but also all finished products including the same.

In addition, the "dispenser device for spraying powder" may be expressed in terms, such as "powder sprayer", "powder spraying device", "powder spray", "powder spray device", etc. If the device is a device used to apply pharmaceutical powder to the human body, it may be interpreted as the "dispenser device for spraying powder".

According to one aspect of the present disclosure, a dispenser device for spraying pharmaceutical powder is provided.

In this specification, the term "pharmaceutical powder" refers to drugs in powder form that are used clinically, and may also be used as expressions, such as "medicinal powder", "medicinal powder body", and "powdered drug". Examples of the pharmaceutical powder include various products, such as hemostatic agents, anti-adhesion agents, wound dressings, and medicinal adhesives, but are not limited thereto, and those skilled in the art will easily understand that various pharmaceutical powders may be included.

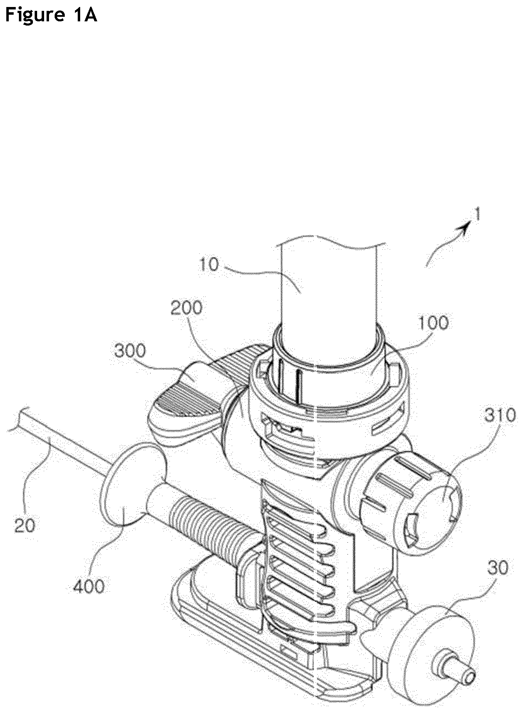

FIG. 1A is a perspective view of a dispenser device for spraying pharmaceutical powder according to an embodiment of the present disclosure.

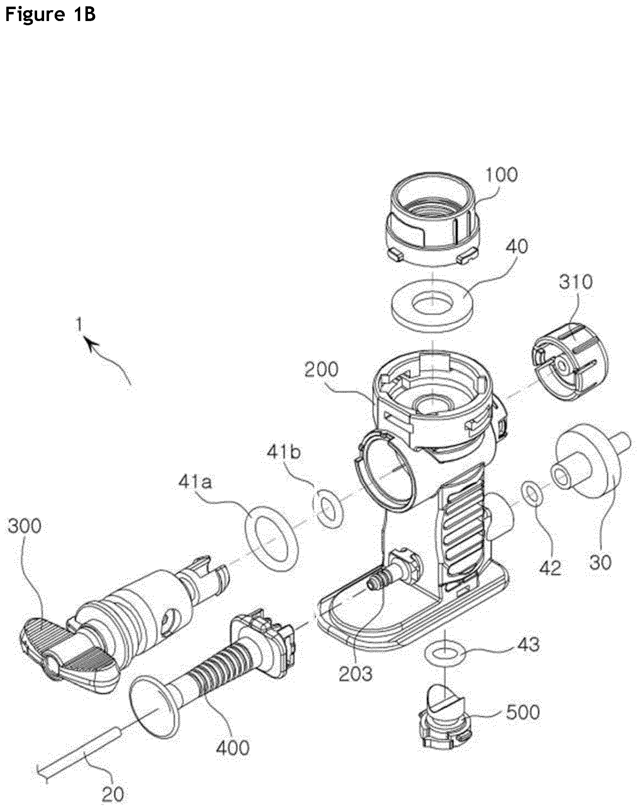

FIG. 1B is an exploded view of the dispenser device for spraying pharmaceutical powder, illustrating each component thereof according to an embodiment of the present disclosure.

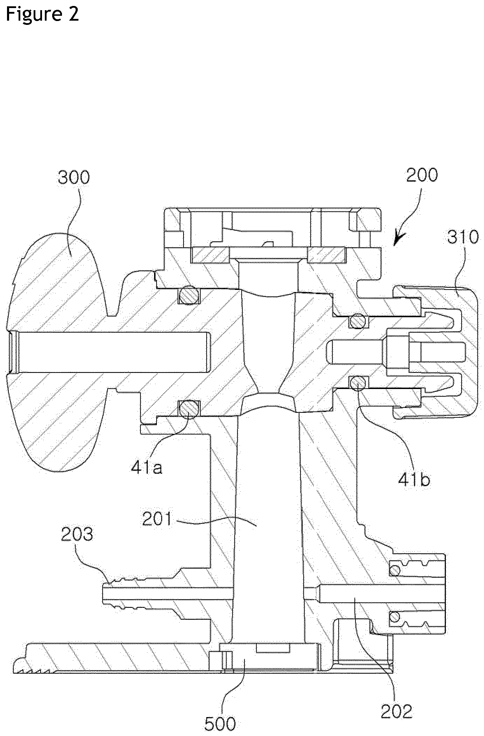

FIG. 2 is a cross-sectional view of a housing according to an embodiment of the present disclosure.

Referring to FIGS. 1A, 1B, and 2, the dispenser device for spraying pharmaceutical powder according to an embodiment of the present disclosure includes: (a) a drug container adapter 100 enabling a pharmaceutical powder container to be mounted thereto; (b) a housing 200; and (c) a control valve 300 for controlling the flow of the pharmaceutical powder.

More specifically, the housing includes: a first transfer tube 201 communicating with the drug container adapter 100to allow the pharmaceutical powder delivered from the pharmaceutical powder container to pass therethrough; and a second transfer tube 202 communicating with the first transfer tube 201 so as to discharge the pharmaceutical powder together with air, the second transfer tube 202 having both ends communicating with an air inlet 204 and a conduit mounting part 203, respectively.

In addition, the control valve 300 is located in the middle of first transfer tube 201 of the housing.

As shown in FIG. 2, in the device of the present disclosure, the first transfer tube 201 of the housing may be communicated in the vertical direction, but is not limited thereto.

In an embodiment of the present disclosure, the valve is fitted into the housing.

In an embodiment of the present disclosure, a packing (e.g., O-ring) is inserted into a gap between the valve and the housing coupled to each other, to prevent leakage of air or pharmaceutical powder. In a specific embodiment of the present disclosure, packings are inserted in the flow path through which the valve and the first transfer tube communicate with each other, and one packing is disposed at each of both the valve side and the first transfer tube side.

In an embodiment of the present disclosure, a hook is formed in the valve to be insertion-coupled (snap-fit) to the housing, and the housing has a locking ledge on which the hook may be hooked and fixed.

In an embodiment of the present disclosure, the dispenser device for spraying pharmaceutical powder may further include a valve cap 310 configured to cover a portion where the hook of the valve and a locking ledge structure of the housing are coupled with each other.

In an embodiment of the present disclosure, the valve may be a ball valve or a butterfly valve having a flow path formed in the valve.

In an embodiment of the present disclosure, the valve controls the flow of pharmaceutical powder and opening/closing thereof by rotating the valve at an angle of 90° or less.

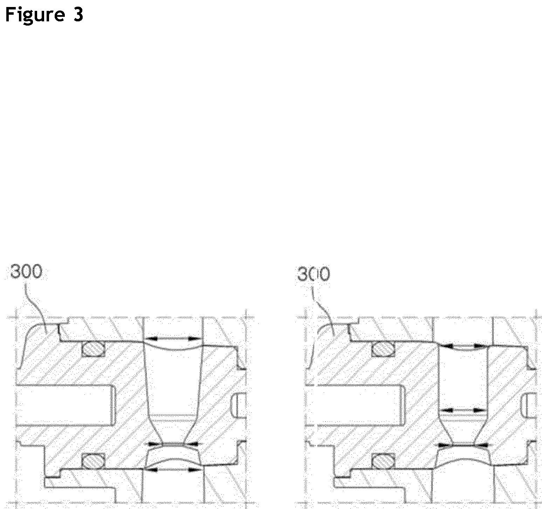

FIG. 3 illustrates a structure in which the diameters of both ends of a flow path in a valve are wider than the diameter of the middle of the flow path to prevent the valve of the present disclosure from becoming inoperable.

Referring to FIG. 3, in a specific embodiment of the present disclosure, when the valve of the present disclosure is in an open state, the inner diameter of the flow path in the valve narrows in the direction in which the pharmaceutical powder passes, and then widens again. Accordingly, the cross section of the flow path of the valve has a shape such as a sagittal cross section of a Venturi tube or an hourglass.

The structure as described above may prevent the operation of the valve from being disturbed due to internal friction caused by the penetration of pharmaceutical powder between the valve and the housing during opening and closing operations of the valve.

In another specific embodiment of the present disclosure, the inner diameter of the valve of the present disclosure may be maintained constant.

In another specific embodiment of the present disclosure, the inner diameter of the valve of the present disclosure may become narrower in the direction in which the pharmaceutical powder passes, and then remain constant.

In a specific embodiment of the present disclosure, the inner diameters of both ends of the flow path of the valve are identical to or similar (±10%) to the inner diameter of the first transfer tube, but are not limited thereto.

When the valve is in an open state, the pharmaceutical powder passes through the first transfer tube and reaches a portion communicating with the second transfer tube, and when the valve is in a closed state, the pharmaceutical powder may be completely blocked from passing through the first transfer tube, and thus, fine spray phenomenon of the pharmaceutical powder may be prevented.

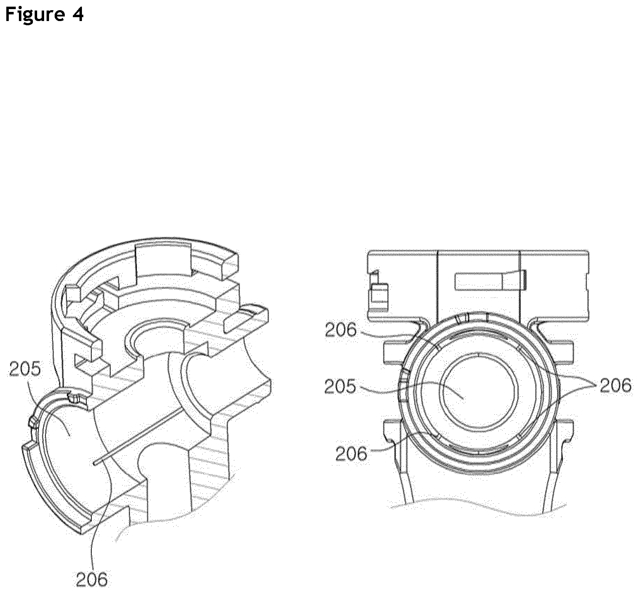

FIG. 4 illustrates a fine protrusion formed to block fine gaps in a portion (a valve rotation part) where the control valve is combined with the housing in the device for spraying pharmaceutical powder according to the present disclosure.

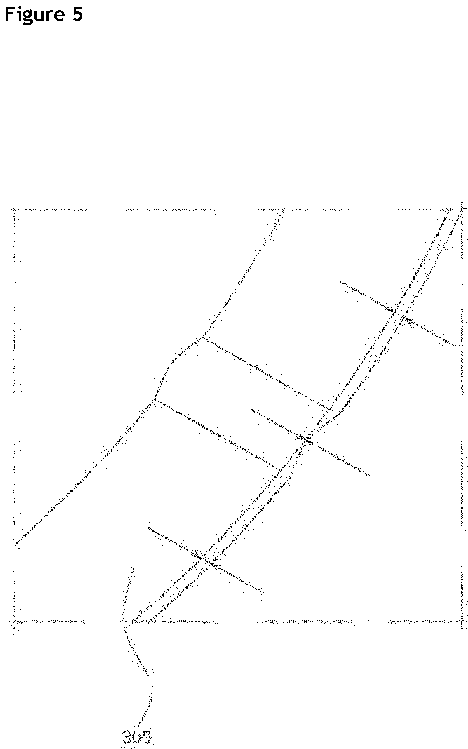

FIG. 5 is an enlarged view of a portion where the valve and the fine protrusion of a valve accommodating part are in contact with each other.

Referring to FIGS. 4 and 5, in an embodiment of the present disclosure, in order to open and close the valve, the fine protrusion 206 may be formed on a portion (the valve accommodating part 205) where the valve is rotated in the housing.

The fine protrusion 206 blocks a fine gap between the valve and the valve accommodating part 205 while maintaining the operability of the valve, thereby blocking the pharmaceutical powder from finely flowing (leaking) through the gap.

In an embodiment of the present disclosure, as the result of applying a circular fine protrusion of 0.05 mm to the place where the housing and the valve are assembled, the leakage of powder through the gap between the housing and the valve was blocked while the operability was maintained.

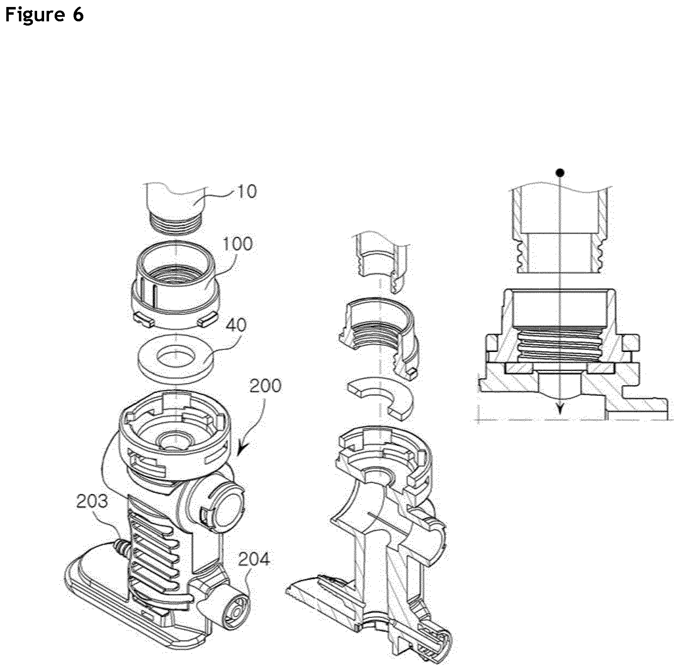

FIG. 6 is a perspective view and a cross-sectional view illustrating a combination of a drug container adapter, a drug container, and the housing according to the present disclosure.

Referring to FIG. 6, in an embodiment of the present disclosure, the drug container may be coupled to the drug container adapter in the vertical direction. As described above, when the drug container is coupled to the drug container adapter or the housing in the vertical direction, the pharmaceutical powder inside the drug container may be more efficiently transferred toward the housing by gravity.

In an embodiment of the present disclosure, the drug container adapter may be insertion-coupled (snap-fit) or screw-coupled to the housing. A sealing packing (i.e., an adapter seal 40) is inserted between the drug container adapter and the housing.

In an embodiment of the present disclosure, the pharmaceutical powder container may be screwed to the drug container adapter, but is not limited thereto.

In an embodiment of the present disclosure, as noted from the result of coupling the drug container adapter having the structure shown in FIG. 6 to the housing together with the adapter seal 40, combining the drug container containing the pharmaceutical powder, and testing for airtightness, the pressure loss was maintained at 0.005 psi or lower for 5 seconds. Therefore, the NBM internal technical selection was satisfied.

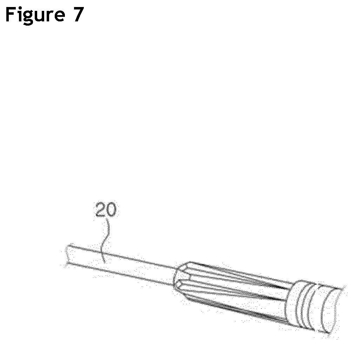

FIG. 7 illustrates the shape of a tube cap configured to guide an existing conduit 20 in general use to a conduit mounting part.

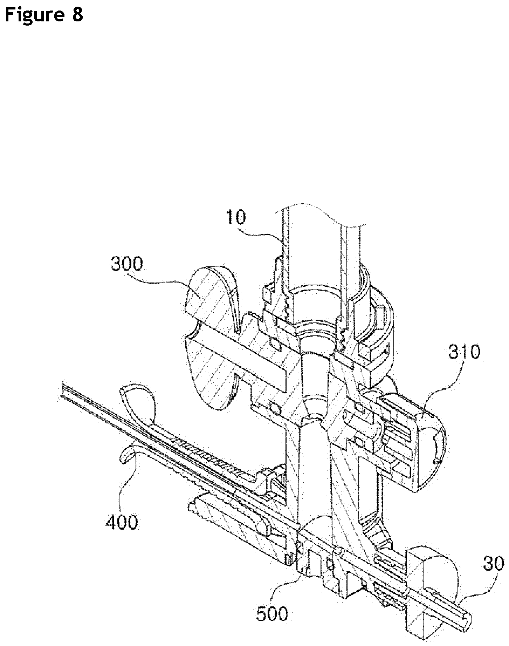

FIG. 8 illustrates a tube cap configured to guide the conduit 20 to the conduit mounting part in order to prevent the conduit 20 from bending, according to the present disclosure.

Referring to FIGS. 7 and 8, in an embodiment of the present disclosure, the device for spraying powder further includes a tube cap 400 coupled in a direction parallel to the second transfer tube.

The tube cap 400 is coupled to the conduit mounting part of the housing, and has a lumen diameter at a distal portion greater than a lumen diameter at a proximal portion, based on the housing.

The tube cap 400 has a lumen diameter at a distal portion greater than a lumen diameter at a proximal portion, based on the housing, and may be thus referred to as a fallopian tube cap due to the trumpet-like shape thereof. The tube cap guides the conduit 20 to be mounted to the conduit mounting part, prevents bending and deformation of the conduit 20 caused by the diameter of the distal part being wider than the diameter of the proximal part, and provides a free range of motion.

In an embodiment of the present disclosure, the tube cap 400 is communicated to enable the conduit 20 to pass therethrough, and guides the conduit to the conduit mounting part 203.

In addition, when the tube cap 400 is coupled to the conduit mounting part of the housing, the conduit mounting part and the tube cap are brought into close contact with each other. When the conduit is pushed into the conduit mounting part through the tube cap to be coupled thereto, the inner diameter of the end of the conduit is increased by the elastic force of the conduit, and the tube cap and the conduit mounting part are in close contact with each other. As a result, the tube cap 400 may also perform a role of maintaining airtightness so that air does not leak while the conduit does not escape out of the conduit mounting part.

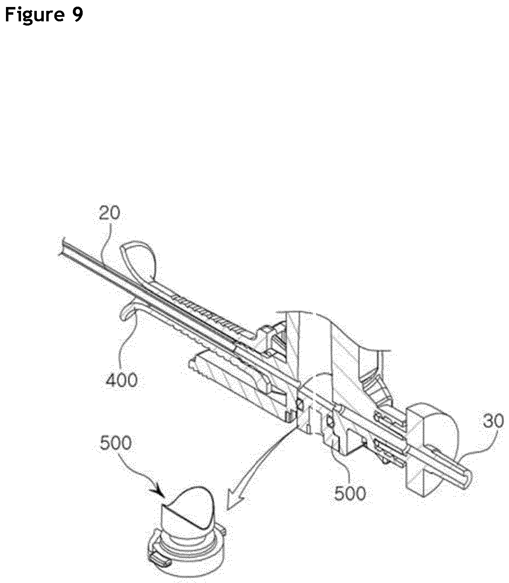

FIG. 9 illustrates the shape of a bottom cap 500 having a groove formed on the bottom surface thereof so that pharmaceutical powder may be collected at a portion where first and second transfer tubes of the present disclosure meet and communicate with each other.

Referring to FIG. 9, in an embodiment of the present disclosure, the bottom cap 500 (bottom cap) having a groove formed on the bottom surface thereof in the direction of air flow is coupled to the portion where the first transfer tube and the second transfer tube meet and communicate with each other. The groove may be formed in a U-shape or V-shape.

In another embodiment of the present disclosure, the bottom cap 500 may be provided at a portion where the first transfer tube and the second transfer tube of the present disclosure meet and communicate with each other. The bottom cap 500 blocks the lower surface of the first transfer tube to enable the pharmaceutical powder falling along the first transfer tube to be moved and sprayed along the air flow direction of the second transfer tube. The shape of the bottom cap 500 may have a flat bottom surface, a round bottom surface, etc., in addition to the above-described U-shape, V-shape, or other grooved shapes, but is not limited thereto.

In an embodiment of the present disclosure, a packing (e.g., O-ring) is inserted into a gap between the bottom cap 500 and the housing coupled to each other, to prevent leakage of air or powder.

As shown in FIG. 9, the inner diameter of the first transfer tube widens toward the second transfer tube. When the inner diameter of the first transfer tube widens toward the second transfer tube as described above, the pharmaceutical powder is attached to the sidewall of the first transfer tube, thereby minimizing the waste thereof.

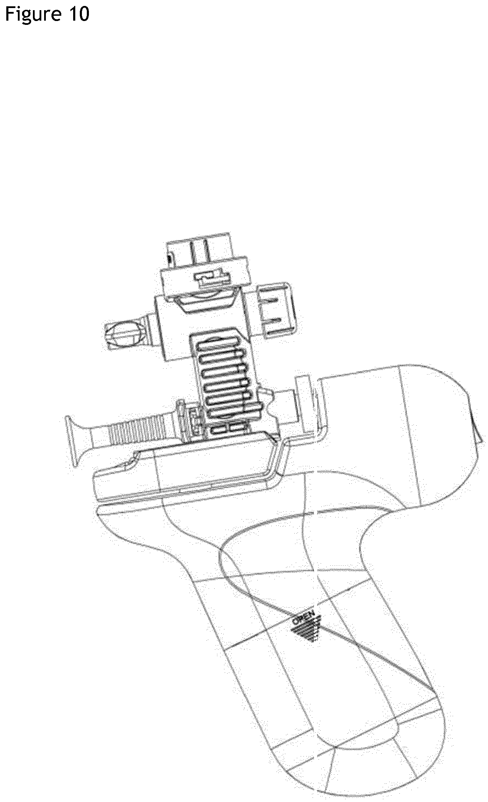

FIG. 10 illustrates an air supply device additionally coupled to the dispenser device for spraying pharmaceutical powder according to the present disclosure.

As shown in FIG. 10, the dispenser device for spraying powder of the present disclosure may further include an air supply device 2 or may be coupled thereto, the air supply device 2 being coupled to the air inlet 204 of the housing.

In addition, in an embodiment of the present disclosure, the device may further include an air filter 30 between the air inlet 204 and the air supply device 2.

In an embodiment of the present disclosure, the air inlet 204 of the housing and the air supply device 2 or the air inlet 204 of the housing and the air filter 30 may be screw-coupled or insertion-coupled (snap-fit) to each other, but is not limited thereto.

In an embodiment of the present disclosure, a packing (e.g., O-ring) is inserted into a gap between the air inlet 204 of the housing and the air supply device 2 coupled to each other or a gap between the air inlet 204 of the housing and the air filter 30 coupled to each other, to prevent leakage of the filtered air.

In addition, according to the dispenser device 1 for spraying pharmaceutical powder according to another aspect of the present disclosure, a dispenser device for spraying pharmaceutical powder, including the elements of the above-described device, is provided.

In an embodiment of the present disclosure, the dispenser device for spraying pharmaceutical powder further includes a vibrator. The vibrator generates vibration to facilitate falling of the drug powder inside the drug container.

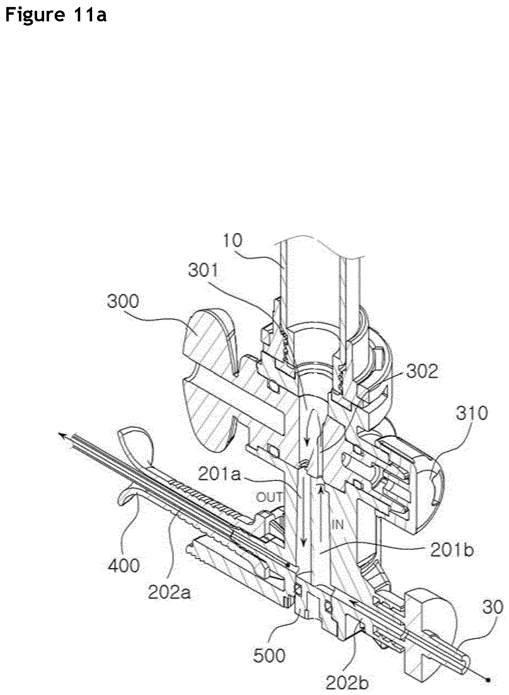

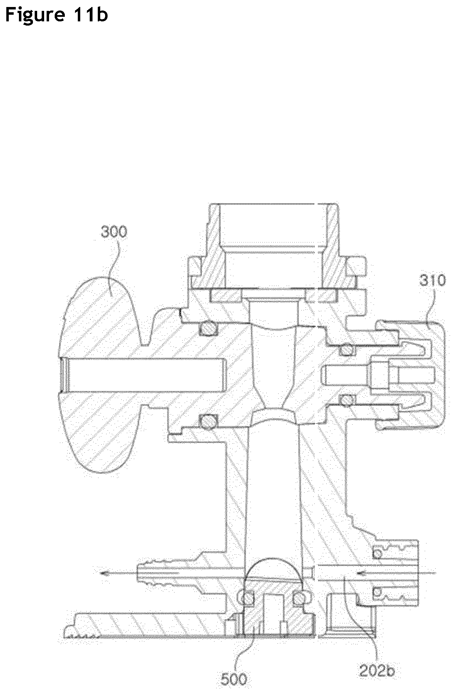

FIG. 11A, which illustrates a dispenser device for spraying pharmaceutical powder in which the first transfer tube and the control valve of the present disclosure have two separate channels, respectively, is a cross-section view of the control valve in an open state, and FIG. 11B is a view showing an orifice structure in which the inner diameter of the channel is narrowed in the direction of air flow in a second transfer tube inlet channel 202b as a modified form of the the second transfer tube.

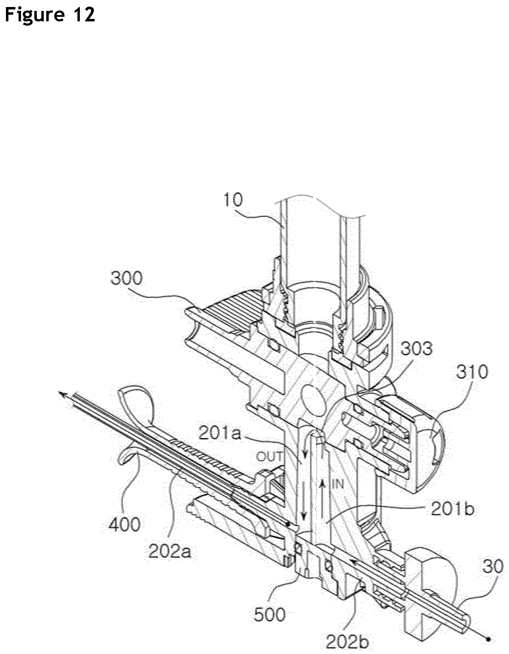

FIG. 12, is a cross-sectional view of the control valve in a closed state.

As shown in FIG. 11A, in the dispenser device for spraying powder of the present disclosure, the first transfer tube 201 includes a first transfer tube upper channel 201b for transferring air flow upward, and a first transfer tube lower channel 201a for transferring air flow downward.

The first transfer tube upper channel 201b according to an embodiment of the present disclosure transfers the air introduced into the housing from the second transfer tube toward the control valve and the drug container. The air transferred toward the control valve and the drug container may be circulated again in the order of the control valve and the first transfer tube lower channel 201b, and accordingly, the drug may be discharged through the second transfer tube.

In an embodiment of the present disclosure, the second transfer tube 202 includes a second transfer tube inlet channel 202b for introducing air into the housing, and a second transfer tube discharge channel 202a for discharging air to the outside of the housing.

In an embodiment of the present disclosure: the first transfer tube 201 includes a first transfer tube upper channel 201b for transferring air flow upward, and a first transfer tube lower channel 201a for transferring air flow downward;
the second transfer tube includes a second transfer tube inlet channel 202b for introducing air into the housing, and a second transfer tube discharge channel 202a for discharging air to the outside of the housing; and
the second transfer tube inlet channel 202b communicates with the first transfer tube upper channel 201b, and the second transfer tube discharge channel 202a communicates with the first transfer tube lower channel 201a.

As in the dispenser device for spraying pharmaceutical powder according to an embodiment of the present disclosure, the air introduced into the second transfer tube inlet channel 202b is transferred toward the control valve and the drug container through the first transfer tube upper channel 201b. The transferred air is moved to the first transfer tube lower channel 201a through the control valve together with the drug in the container. The air transferred through the first transfer tube lower channel 201a is sprayed out of the device together with the drug through the second transfer tube discharge channel 202a.

In addition, as shown in FIG. 11B, in another embodiment of the present disclosure, the second transfer tube inlet channel 202b may have an orifice structure in which the inner diameter of the channel narrows in the air flow direction. As described above, when the inner diameter of the channel is narrowed in the direction of air flow, the flow rate can be increased even at the same inlet pressure.

In an embodiment of the present disclosure, the control valve includes a control valve upper channel 302 for transferring air flow upward, and a control valve lower channel 301 for transferring air flow downward.

In a specific embodiment of the present disclosure, in an open state, the control valve upper channel 302 communicates with the first transfer tube upper channel 201b, and the control valve lower channel 301 communicates with the first transfer tube lower channel 201a.

In another specific embodiment of the present disclosure, the inner diameter of the control valve lower channel 301 narrows toward a portion communicating with the first transfer tube lower channel 201a while the control valve is open.

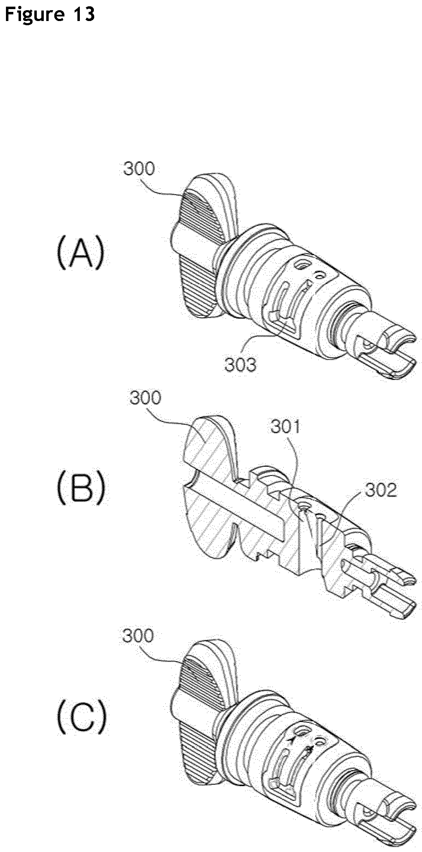

In still another specific embodiment of the present disclosure, the control valve of the present disclosure is provided with an air closure prevention notch 303. The air closure prevention notch 303 communicates the first transfer tube upper channel 201b with the first transfer tube lower channel 201a due to the recessed structure thereof when the control valve is closed. The specific structure of the air closure prevention notch 303 is shown in FIGS. 12 and 13.

When the control valve is closed, the air introduced from the first transfer tube upper channel 201b is not transferred to the drug container. However, even when the control valve is closed, the air introduced from the first transfer tube upper channel 201b passes through the air closure prevention notch 303 and moves to the first transfer tube lower channel 201a to be discharged outside the device.

A circulation path in which air is directly connected in the order of the first transfer tube upper channel, the control valve, and the first transfer tube lower channel is referred to as an air idle pathway.

On the other hand, a circulation path in which air is connected in the order of the first transfer tube upper channel, the control valve, the drug container, the control valve, and the first transfer tube lower channel under the condition that the control valve is open is referred to as an air through pathway.

FIG. 13 illustrates a three-dimensional structure (A, C) and a cross section (B) of the control valve having two separate channels of FIG. 12 in a closed state. The air closure prevention notch 303 is configured to maintain a circulation path of air even when the control valve is switched from an open state to a closed state, and thus extends along the surface periphery of the control valve in the rotation direction. As a result, as shown in given reference A in FIG. 13, the air closure prevention notch 303 may be configured in a 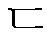 -shape on the surface of the control valve.

The air closure prevention notch 303 is disconnected from the control valve upper channel 302, and the control valve lower channel 301 for transferring air flow downward.

According to another aspect of the present disclosure, the present disclosure provides a method of using the dispenser device for spraying pharmaceutical powder, the method including the following operations of:
(i) coupling the air supply device 2 to the air inlet 204 of the dispenser device for spraying pharmaceutical powder;
(ii) coupling the tube cap 400 and the conduit to the conduit mounting part 203;
(iii) coupling a container containing pharmaceutical powder to the drug container adapter 100;
(iv) turning on the powder of the air supply device 2; and
(v) controlling the opening/closing state of the control valve and spraying the pharmaceutical powder.

In an embodiment of the present disclosure, the order of operations (i) to (iii) may be changed.

In another embodiment of the present disclosure, the tube cap has a lumen diameter at a distal portion greater than a lumen diameter at a proximal portion, based on the housing.

Since the method of using the dispenser device for spraying powder according to one aspect of the present disclosure relates to the method of using the dispenser device for spraying powder according to one aspect of the present disclosure including the above-described elements, the same description applies to the same elements between both disclosures. Therefore, to avoid the complexity of the present specification, the description of the same elements will be omitted without being provided again.

In the above, the present disclosure has been described in detail with reference to the drawings together with preferred embodiments, but the scope of the technical idea of the present disclosure is not limited to these drawings and embodiments. Therefore, various modified examples or embodiments of equivalent range may exist within the scope of the technical idea of the present disclosure. Accordingly, the scope of the technical idea according to the present disclosure should be interpreted by the claims, and the technical idea within the equivalent or equivalent range should be construed as belonging to the scope of the present disclosure.

### [Description of Symbols]

1: Dispenser device for spraying pharmaceutical powder
2: Air supply device
10: Drug container
20: Conduit
30: Air filter
40: Adapter seal
41a, 41b, 42, 43: O-ring
100: Drug container adapter
200: Housing
201: First transfer tube
201a: First transfer tube lower channel
201b: First transfer tube upper channel
202: Second transfer tube
202a: Second transfer tube discharge channel
202b: Second transfer tube inlet channel
203: Conduit mounting part
204: Air inlet
205: Valve accommodating part
206: Fine protrusion
300: Control valve
301: Control valve lower channel
302: Control valve upper channel
303: Air closure prevention notch
310: Valve cap
400: Tube cap
500: Bottom cap

## Claims

1. A dispenser device (1) for spraying pharmaceutical powder, comprising:
(a) a drug container adapter (100) enabling a pharmaceutical powder container to be mounted thereto;
(b) a housing (200) comprising a first transfer tube (201) vertically communicating with the drug container adapter (100) to allow pharmaceutical powder delivered from a pharmaceutical powder container to pass downward therethrough, and a second transfer tube (202) crossing the first transfer tube (201) and communicating with the first transfer tube (201) so as to discharge the pharmaceutical powder together with air, the second transfer tube (202) having both ends communicating with a conduit mounting part (203) configured to receive a conduit, and an air inlet (204), configured to be connected to an air supply device (2) respectively; and
(c) a control valve (300) located between the drug container adapter (100) and the first transfer tube to control the flow of the pharmaceutical powder, wherein:
the first transfer tube (201) comprises a first transfer tube upper channel (201b) configured to transfer air flow upward, and a first transfer tube lower channel (201a) configured to transfer air flow downward;
the second transfer tube (202) comprises a second transfer tube inlet channel (202b) configured to introduce air into the housing, and a second transfer tube discharge channel (202a) configured to discharge air to the outside of the housing;
the second transfer tube inlet channel (202b) communicates with the first transfer tube upper channel (201b), and the second transfer tube discharge channel (202a) communicates with the first transfer tube lower channel (201a); the control valve (300) is a ball valve or a butterfly valve having a flow path formed in the valve;
the control valve (300) comprises a control valve upper channel (302) configured to transfer air flow upward, and a control valve lower channel (301) configured to transfer air flow downward;
the control valve upper channel (302) and the control valve lower channel (301) are in communication with the first transfer tube upper channel (201b) and first transfer tube lower channer (201a), respectively;
the control valve is provided with an air closure prevention notch (303) having a recessed structure which communicates the first transfer tube upper channel (201b) with the first transfer tube lower channel (201a) when the control valve is closed, such that the air introduced from the first transfer tube upper channel (201b) is not transferred to the drug container but passes through the air closure prevention notch (303) and moves to the first transfer tube lower channel (201a); and
the control valve (300) is positioned above the intersection of the first transfer tube (201) and the second transfer tube (202), such that when the control valve (300) is in a closed state, air entering through the air inlet (204) is prevented from flowing beyond the upper section of the control valve (300).

2. The dispenser device (1) of claim 1, wherein when the control valve (300) is open, an inner diameter of a flow path in the control valve i) is constant, ii) narrows in the direction in which the pharmaceutical powder passes and then widens again, or iii) becomes narrower in the direction in which the pharmaceutical powder passes and then remains constant.

3. The dispenser device (1) of claim 1, wherein in order to open and close the valve, a fine protrusion (206) is formed on a valve accommodating part (205) where the valve is rotated in the housing (200).

4. The dispenser device (1) of claim 1, further comprising a tube cap (400) coupled to the conduit mounting part (203) wherein the tube cap (400) has an inner lumen diameter that increases from the conduit mounting portion of the housing towards the distal end.

5. The dispenser device (1) of claim 1, wherein a bottom cap (500) is coupled to a portion where the first transfer tube (201) and the second transfer tube (202) communicate with each other.

6. The dispenser device (1) of claim 1, wherein an inner diameter of the first transfer tube (201) widens toward the second transfer tube (202).

7. The dispenser device (1) of claim 1, further comprising an air supply device (2) coupled to the air inlet (204) of the housing (200).

8. A method of using the dispenser device (1) of any of the preceding claims for spraying pharmaceutical powder, the method comprising:
(i) coupling an /the air supply device (2) to the air inlet (204) of the dispenser device (1) for spraying pharmaceutical powder of claim 1;
(ii) coupling a/ the tube cap (400) and a conduit to the conduit mounting part (203);
(iii) coupling a container containing pharmaceutical powder to the drug container adapter (100);
(iv) turning on the power of the air supply device (2); and
(v) controlling the opening/closing state of the control valve (300) and spraying the pharmaceutical powder,
wherein the tube cap (400) has an inner lumen diameter that increase from the conduit mounting part (203) of the housing toward the distal end.

9. The method of claim 8, wherein the order of operations (i) to (iii) is changeable.

## Patentansprüche

1. Abgabevorrichtung (1) zum Sprühen von pharmazeutischem Pulver, umfassend:
(a) einen Arzneimittelbehälteradapter (100), der ermöglicht, dass ein Behälter für pharmazeutisches Pulver daran montiert wird;
(b) ein Gehäuse (200), umfassend eine erste Übertragungsröhre (201), die mit dem Arzneimittelbehälteradapter (100) vertikal kommuniziert, um zuzulassen, dass pharmazeutisches Pulver, das von einem Behälter für pharmazeutisches Pulver zugeführt wird, dadurch hindurch passiert, und eine zweite Übertragungsröhre (202), die die erste Übertragungsröhre (201) kreuzt und mit der ersten Übertragungsröhre (201) kommuniziert, um das pharmazeutische Pulver zusammen mit Luft zu entladen, wobei bei der zweiten Übertragungsröhre (202) beide Enden mit einem Leitungsmontageteil (203) kommunizieren, das konfiguriert ist, um eine Leitung aufzunehmen, und einen Lufteinlass (204), der konfiguriert ist, um mit einer Luftzufuhrvorrichtung (2) jeweils verbunden zu sein; und
(c) ein Steuerventil (300), das sich zwischen dem Arzneimittelbehälteradapter (100) und der ersten Übertragungsröhre befindet, um den Fluss des pharmazeutischen Pulvers zu steuern, wobei:
die erste Übertragungsröhre (201) einen ersten oberen Übertragungsröhrenkanal (201b), der konfiguriert ist, um Luftstrom nach oben zu übertragen, und einen ersten unteren Übertragungsröhrenkanal (201a) umfasst, der konfiguriert ist, um Luftstrom nach unten zu übertragen;
die zweite Übertragungsröhre (202) einen zweiten Übertragungsröhren-Einlasskanal (202b), der konfiguriert ist, um Luft in das Gehäuse einzuführen, und einen zweiten Übertragungsröhren-Auslasskanal (202a) umfasst, der konfiguriert ist, um Luft außerhalb des Gehäuses abzulassen;
der zweite Übertragungsröhren-Einlasskanal (202b) mit dem ersten oberen Übertragungsröhrenkanal (201b) kommuniziert und der zweite Übertragungsröhren-Auslasskanal (202a) mit dem ersten unteren Übertragungsröhrenkanal (201a) kommuniziert;
das Steuerventil (300) ein Kugelventil oder ein Schmetterlingsventil mit einem Strömungsweg ist, der im Ventil gebildet ist;
das Steuerventil (300) einen oberen Steuerventilkanal (302) umfasst, der konfiguriert ist, um Luftstrom nach oben zu übertragen, und einen unteren Steuerventilkanal (301) umfasst, der konfiguriert ist, um Luftstrom nach unten zu übertragen;
der obere Steuerventilkanal (302) und der untere Steuerventilkanal (301) in Kommunikation mit dem ersten oberen Übertragungsröhrenkanal (201b) bzw. dem ersten unteren Übertragungsröhrenkanal (201a) in Kommunikation sind;
das Steuerventil mit einem Luftverschluss-Präventionskerbe (303) bereitgestellt ist, die eine vertiefte Struktur aufweist, die den ersten oberen Übertragungsröhrenkanal (201b) mit dem ersten unteren Übertragungsröhrenkanal (201a) kommuniziert, wenn das Steuerventil verschlossen ist, sodass die Luft, die von dem ersten oberen Übertragungsröhrenkanal (201b) eingeführt wird, nicht zum Arzneimittelbehälter übertragen wird, sondern durch die Luftverschluss-Präventionskerbe (303) passiert und sich zum ersten unteren Übertragungsröhrenkanal (201a) bewegt; und
das Steuerventil (300) über dem Schnittpunkt der ersten Übertragungsröhre (201) und der zweiten Übertragungsröhre (202) positioniert ist, sodass, wenn das Steuerventil (300) in einem verschlossenen Zustand ist, verhindert wird, dass Luft, die durch den Lufteinlass (204) eintritt, über den oberen Abschnitt des Steuerventils (300) hinweg strömt.

2. Abgabevorrichtung (1) nach Anspruch 1, wobei, wenn das Steuerventil (300) geöffnet ist, ein Innendurchmesser eines Strömungswegs im Steuerventil i) konstant ist, ii) sich in die Richtung, in die das pharmazeutische Pulver passiert, verengt und sich dann wieder erweitert, oder iii) enger in der Richtung wird, in die das pharmazeutische Pulver passiert, und dann konstant bleibt.

3. Abgabevorrichtung (1) nach Anspruch 1, wobei, um das Ventil zu öffnen und zu verschließen, ein feiner Vorsprung (206) an einem Ventilaufnahmeteil (205) gebildet ist, wo das Ventil im Gehäuse (200) gedreht wird.

4. Abgabevorrichtung (1) nach Anspruch 1, ferner umfassend eine Röhrenkappe (400), die an das Leitungsmontageteil (203) gekoppelt ist, wobei die Röhrenkappe (400) einen Innenlumendurchmesser aufweist, der von dem Leitungsmontageabschnitt des Gehäuses zum distalen Ende zunimmt.

5. Abgabevorrichtung (1) nach Anspruch 1, wobei eine Bodenkappe (500) an einen Abschnitt gekoppelt ist, wo die erste Übertragungsröhre (201) und die zweite Übertragungsröhre (202) miteinander kommunizieren.

6. Abgabevorrichtung (1) nach Anspruch 1, wobei sich ein Innendurchmesser der ersten Übertragungsröhre (201) zur zweiten Übertragungsröhre (202) hin erweitert.

7. Abgabevorrichtung (1) nach Anspruch 1, ferner umfassend eine Luftzufuhrvorrichtung (2), die an den Lufteinlass (204) des Gehäuses (200) gekoppelt ist.

8. Verfahren des Verwendens der Abgabevorrichtung (1) nach einem der vorhergehenden Ansprüche zum Sprühen von pharmazeutischem Pulver, wobei das Verfahren Folgendes umfasst:
(i) Koppeln einer/der Luftzufuhrvorrichtung (2) mit dem Lufteinlass (204) der Abgabevorrichtung (1) zum Sprühen von pharmazeutischem Pulver nach Anspruch 1;
(ii) Koppeln einer/der Röhrenkappe (400) und einer Leitung mit dem Leitungsmontageteil (203);
(iii) Koppeln eines Behälters, der pharmazeutisches Pulver enthält, mit dem Arzneimittelbehälteradapter (100);
(iv) Einschalten des Stroms der Luftzufuhrvorrichtung (2); und
(v) Steuern des Öffnungs-/Verschlusszustands des Steuerventils (300) und Sprühen des pharmazeutischen Pulvers,
wobei die Röhrenkappe (400) einen Innenlumendurchmesser aufweist, der vom Leitungsmontageteil (203) des Gehäuses zum distalen Ende zunimmt.

9. Verfahren nach Anspruch 8, wobei die Reihenfolge der Vorgänge (i) bis (iii) veränderbar ist.

## Revendications

1. Dispositif distributeur (1) pour pulvériser une poudre pharmaceutique, comprenant :
(a) un adaptateur pour récipient de médicament (100) permettant de monter un récipient de poudre pharmaceutique sur celui-ci,
(b) un boîtier (200) comprenant un premier tube de transfert (201) communiquant verticalement avec l'adaptateur pour récipient de médicament (100) pour permettre à une poudre pharmaceutique issue d'un récipient de poudre pharmaceutique de descendre à travers celui-ci, et un deuxième tube de transfert (202) croisant le premier tube de transfert (201) et communiquant avec le premier tube de transfert (201) de façon à éjecter la poudre pharmaceutique conjointement avec de l'air, les deux extrémités respectives du deuxième tube de transfert (202) étant en communication avec une partie de montage de conduite (203) conçue pour recevoir une conduite, et une entrée d'air (204) conçue pour être raccordée à un dispositif d'apport d'air (2), et
(c) une vanne de régulation (300) située entre l'adaptateur pour récipient de médicament (100) et le premier tube de transfert pour réguler le passage de la poudre pharmaceutique ; étant entendu que :
le premier tube de transfert (201) comprend un canal supérieur de premier tube de transfert (201b) conçu pour transférer un flux d'air vers le haut, et un canal inférieur de premier tube de transfert (201a) conçu pour transférer un flux d'air vers le bas,
le deuxième tube de transfert (202) comprend un canal d'entrée de deuxième tube de transfert (202b) conçu pour introduire de l'air dans le boîtier, et un canal d'éjection de deuxième tube de transfert (202a) conçu pour éjecter de l'air à l'extérieur du boîtier,
le canal d'entrée de deuxième tube de transfert (202b) communique avec le canal supérieur de premier tube de transfert (201b), et le canal d'éjection de deuxième tube de transfert (202a) communique avec le canal inférieur de premier tube de transfert (201a),
la vanne de régulation (300) est un robinet à bille ou un robinet à papillon dans lequel est formée une voie d'écoulement,
le vanne de régulation (300) comprend un canal supérieur de vanne de régulation (302) conçu pour transférer un flux d'air vers le haut, et un canal inférieur de vanne de régulation (301) conçu pour transférer un flux d'air vers le bas,
le canal supérieur de vanne de régulation (302) et le canal inférieur de vanne de régulation (301) communiquent respectivement avec le canal supérieur de premier tube de transfert (201b) et le canal inférieur de premier tube de transfert (201a),
la vanne de régulation est pourvue d'une encoche de prévention de blocage d'air (303) présentant une structure en creux qui met en communication le canal supérieur de premier tube de transfert (201b) avec le canal inférieur de premier tube de transfert (201a) lorsque la vanne de régulation est fermée, de façon que l'air introduit par le canal supérieur de premier tube de transfert (201b) n'est pas transféré vers le récipient de médicament mais passe à travers l'encoche de prévention de blocage d'air (303) et passe vers le canal inférieur de premier tube de transfert (201a), et
la vanne de régulation (300) est positionnée au-dessus de l'intersection du premier tube de transfert (201) et du deuxième tube de transfert (202), de façon que lorsque la vanne de régulation (300) est dans un état fermé, l'air entrant par l'entrée d'air (204) est empêché de circuler au-delà de la section supérieure de la vanne de régulation (300).

2. Dispositif distributeur (1) selon la revendication 1, dans lequel, lorsque la vanne de régulation (300) est ouverte, le diamètre intérieur d'une voie d'écoulement dans la vanne de régulation i) est constant, ii) rétrécit dans le sens dans lequel passe la poudre pharmaceutique, puis s'élargit de nouveau, ou iii) devient plus étroit dans le sens dans lequel passe la poudre pharmaceutique, puis reste constant.

3. Dispositif distributeur (1) selon la revendication 1, dans lequel, pour permettre l'ouverture et la fermeture de la vanne, une fine saillie (206) est formée sur une partie de logement de vanne (205) où la vanne tourne dans le boîtier (200).

4. Dispositif distributeur (1) selon la revendication 1, comprenant en outre un capuchon de tube (400) couplé à la partie de montage de conduite (203), le capuchon de tube (400) présentant un diamètre de lumière intérieure qui augmente à partir de la partie de montage de conduite du boîtier vers l'extrémité distale.

5. Dispositif distributeur (1) selon la revendication 1, dans lequel un capuchon inférieur (500) est couplé à une partie où le premier tube de transfert (201) et le deuxième tube de transfert (202) communiquent entre eux.

6. Dispositif distributeur (1) selon la revendication 1, dans lequel le diamètre intérieur du premier tube de transfert (201) s'élargit vers le deuxième tube de transfert (202).

7. Dispositif distributeur (1) selon la revendication 1, comprenant en outre un dispositif d'apport d'air (2) couplé à l'entrée d'air (204) du boîtier (200).

8. Procédé d'utilisation du dispositif distributeur (1) selon l'une quelconque des revendications précédentes pour pulvériser une poudre pharmaceutique, le procédé comprenant :
(i) le couplage d'un/du dispositif d'apport d'air (2) à l'entrée d'air (204) du dispositif distributeur (1) pour pulvériser une poudre pharmaceutique selon la revendication 1,
(ii) le couplage d'un/du capuchon de tube (400) et d'une conduite à la partie de montage de conduite (203),
(iii) le couplage d'un récipient contenant une poudre pharmaceutique à l'adaptateur pour récipient de médicament (100),
(iv) la mise sous tension du dispositif d'apport d'air (2), et
(v) la commande de l'état d'ouverture/fermeture de la vanne de régulation (300) et la pulvérisation de la poudre pharmaceutique ;
ledit capuchon de tube (400) présentant un diamètre intérieur de lumière qui augmente à partir de la partie de montage de conduite (203) du boîtier vers l'extrémité distale.

9. Procédé selon la revendication 8, dans lequel l'ordre des opérations (i) à (iii) est modifiable.
